(19) **Europäisches Patentamt / European Patent Office / Office européen des brevets**

(11) **EP 4 379 656 B1**

(12) **EUROPEAN PATENT SPECIFICATION**

(45) Date of publication and mention
of the grant of the patent:
**07.08.2024 Bulletin 2024/32**

(51) International Patent Classification (IPC):
**G06T 7/10** (2017.01)   **G06T 15/08** (2011.01)
**G16H 30/40** (2018.01)   **G16H 40/67** (2018.01)
**G06F 7/483** (2006.01)

(21) Application number: **22210687.4**

(22) Date of filing: **30.11.2022**

(52) Cooperative Patent Classification (CPC):
**G16H 40/67; G16H 30/40;** G06F 7/483

(54) **SYSTEM AND METHOD FOR SEGMENTATION NETWORK SYNCHRONIZATION**

SYSTEM UND VERFAHREN ZUR SEGMENTIERUNGSNETZWERKSYNCHRONISATION

SYSTÈME ET PROCÉDÉ DE SYNCHRONISATION DE RÉSEAU DE SEGMENTATION

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**05.06.2024 Bulletin 2024/23**

(73) Proprietor: **Specto Medical AG**
**4051 Basel (CH)**

(72) Inventors:
• **Faludi, Balázs**
**4056 Basel (CH)**
• **Zentai, Norbert**
**4056 Basel (CH)**
• **Zelechowski, Marek**
**4056 Basel (CH)**
• **Griessen, Mathias**
**3600 Thun (CH)**
• **Cattin, Philippe**
**5210 Windish (CH)**

(74) Representative: **Bittner, Peter et al**
**Peter Bittner und Partner
Herrenwiesenweg 2
69207 Sandhausen (DE)**

(56) References cited:
**US-A1- 2020 054 398    US-A1- 2022 346 888**

• **CHHEANG VUTHEA ET AL: "A collaborative
virtual reality environment for liver surgery
planning", COMPUTERS AND GRAPHICS,
ELSEVIER, GB, vol. 99, 16 July 2021 (2021-07-16),
pages 234 - 246, XP086821022, ISSN: 0097-8493,
[retrieved on 20210716], DOI:
10.1016/J.CAG.2021.07.009**
• **BEICHEL REINHARD ET AL: "Liver segmentation
in contrast enhanced CT data using graph cuts
and interactive 3D segmentation refinement
methods", MEDICAL PHYSICS, AIP, MELVILLE,
NY, US, vol. 39, no. 3, 1 March 2012 (2012-03-01),
pages 1361 - 1373, XP012160890, ISSN:
0094-2405, [retrieved on 20120221], DOI:
10.1118/1.3682171**

## Description

## Technical Field

The present invention generally relates to 3D rendering, and more particularly, relates to the replication of 3D label masks on multiple computing devices in a network.

## Background

Typically, multi-user applications support collaborative sessions which allow connecting front-end devices of multiple users over a network. The connecting can be established either via local area networks, e.g., multiple connected computing devices (e.g., VR stations) at the same physical location, or over a wide area network (e.g., Internet) connection. In a collaborative session, connected users share a virtual environment. A virtual environment includes volumetric representations of items by data objects which are visualized on display devices with the possibility for connected users to interact. Actions performed by a particular user affecting the virtual environment need to be replicated (or synchronized) to the front-end devices of the other users so that each user finally can see the same state of the virtual environment.

In general, there are two approaches to achieving such state replications:

a) Synchronization of the state of the virtual environment. This involves periodically transmitting and updating the relevant data. The term "synchronization" of an application state on multiple devices is used herein as a synonym for the "replication" of such state on said devices and not in the meaning of a time-based synchronization of signals. Usually, one user's device (or a separate, dedicated server in a server farm) acts as the host of a multi-user session. Each user's application transmits the user's inputs to the host, e.g. "I want to grab this object" or "I want to move this object upwards". The host updates the state in its local copy of the virtual environment and periodically sends up-to-date state information to every connected user, e.g., "Object A is at position X" or "User 1 is holding Object B". Each user's application on the respective front-end device uses this information to update the respective local virtual environment state.

b) Synchronization of the user inputs. This approach is similar to a) in that each user transmits respective inputs to the host. However, using approach b), the host does not update a local copy of the virtual environment state. Instead, it simply forwards each user's input to all other connected users. Special care has to be taken to ensure that all connected users receive and process exactly the same sequence of inputs. Each user's application can then use this information to replicate the events represented by the received data in the respective local application on the user's front-end device.

In a), even if the virtual environment state between different users gets out of sync (e.g., one user sees an object at location X, while another user sees the same object at a slightly different location), the states will always get back in sync after a short period of time (typically in the order of a second) because the host periodically sends the same, authoritative, version of the virtual environment state to every user.

With b), the virtual environment state is not explicitly synchronized during a collaborative session (only when initializing a user joining the collaborative session). Only the user inputs are synchronized. Therefore, changes to the relevant state of the virtual environment have to be done in a perfectly deterministic way. In other words, given the same list of user inputs, every user's local application has to arrive at exactly the same virtual environment state. Otherwise, the state will get out of sync, more and more over time.

Synchronizing the state works well if the state is not very complicated. Thereby, the complexity of a state refers to the amount of data needed to store and transmit the state. Approach a) may therefore be used by collaborative applications with relatively simple (low-complexity) states. For example, an online whiteboard application has typically a number of movable objects in the order of 50. In such a case, the entire state fits into a couple of hundreds of bytes. However, in collaborative sessions with huge amounts of data (e.g., the state of thousands of objects, or a few very complex objects), approach a) is not an option because the amount of data specifying the entire virtual environment state is too large to be transmitted quickly enough over the network so that real-time collaboration of the connected users is supported. An update in such high-complexity state applications would induce wait periods lasting for multiple seconds after each update for the respective users which is not acceptable for real-time collaboration scenarios.

Approach b) is usually used by applications where each user is interacting with one (or a few) objects in the virtual environment. In this case, application developers do not have to worry about achieving perfectly deterministic behavior and it is typically sufficient to transmit the positions of a few objects of in the virtual environment. The position and orientation of an object in 3D space can easily be represented in 24 bytes or fewer.

However, the state synchronization approach b) becomes impossible when the amount of data needed to represent the state is too large to be efficiently transmitted over the network used by the collaborative session in real-time. For example, for applications with thousands of objects or objects whose state representation requires a huge amount of data (high-complexity states), the data required to describe the state of all objects quickly exceeds the amount of data that can be reliably transmitted at the desired update rate (typically 10-60 Hz). Another example relates to medical image processing where segmentation techniques are used to collaboratively label voxels of volumetric medical images. That is, the segmentation labels which are applied by one user need to be visible for all connected users in the collaborative session. In particular for such medical applications, exact replication of the respective state to all connected users is mandatory. However, the amount of data required to store the assigned labels can easily exceed what can reliably be transmitted at the desired update rate.

Examples of prior art solutions are described in the following documents. In US patent application US2022346888A1, a system is disclosed for multidimensional data visualization and interaction in an augmented reality, virtual reality, or mixed reality environment. In a medical environment, the disclosed embodiments provide a tool for a doctor, physician, or other medical technician to quickly load and review patient scans in an AR/VR/MR environment. US patent application US2020054398A1 discloses methods for presenting medical imaging data in an interactive virtual environment reality. A system comprises a head mounted display (HMD) that: determines that a transparent 3D object overlaps a 3D model of a portion of anatomy based on a medical imaging scan; sets values for pixels corresponding to portions of the 3D model not occluded by the transparent object by performing a shading operation; sets values for pixels corresponding to portions of the 3D model at the boundary of the transparent object to intensity values taken from the medical imaging data; displays the 3D model with an exterior surface shaded to evoke a 3D object, and internal surfaces at the boundaries of the transparent object not shaded to preserve details embedded in the medical imaging data. The paper "A Collaborative Virtual Reality Environment for Liver Surgery Planning" by Chheang Vuthea et al., July 2021, Computers & Graphics 99(6), discloses a surgical planning software used in the treatment of tumor diseases. A collaborative virtual reality environment is presented to assist liver surgeons in tumor surgery planning to improve virtual resection planning between surgeons in a remote or co-located environment. The system allows surgeons to define and adjust virtual resections on patient-specific organ 3D surfaces and 2D image slices. Changes on both modalities are synchronized, which will enable surgeons to iterate and refine the resection surfaces quickly. The paper "Liver segmentation in contrast enhanced CT data using graph cuts and interactive 3D segmentation refinement methods" by Beichel Reinhard et al., Med Phys. 2012 Mar; 39(3): 1361-1373, published online 2012 Feb 21, evaluates a new approach for liver segmentation. A graph cuts segmentation method is combined with a three-dimensional virtual reality based segmentation refinement approach. The developed interactive segmentation system allows the user to manipulate volume chunks and/or surfaces instead of 2D contours in cross-sectional images.

**Summary**

There is therefore a need to provide a method and a system for efficient state replication to connected devices in a collaborative session, in particular with regard to the state of volumetric data which are to be processed in real-time by the connected devices. The term "real-time" as used throughout this document is understood by a person skilled in the art of digital data processing as "near real-time" or "quasi real-time". Any digital data processing step (e.g., an image processing step) applied to data captured from the real world (e.g., sensor data to be transferred into visual representations of such sensor data) always results in a delay imposed by the digital data processing steps. For convenience, only the term "real-time" will be used throughout this document when referring to such "near real-time" behavior of the disclosed methods and systems.

In particular, the herein disclosed approach is useful for medical image processing applications where segmentation is used to label different organs or pathologies within a 3D dataset. For example, the label information can be used to hide, show or highlight specific organs or regions. When segmenting a volumetric image (e.g., a CT scan), the respective label mask is volumetric, too, so that a label can be assigned to every voxel of the 3D dataset. In general, segmentation in image processing refers to assigning labels to individual 2D pixels in 2D or 3D voxels in 3D. The labels are stored in a separate image data structure which is referred to as label mask herein. Each pixel/voxel of the mask stores a label index of the assigned label. The label index '0' may be used to indicate that no label is assigned to the respective voxel. Typically, the resolution of the label mask is the same as the resolution of the corresponding volumetric image (3D data set), but this is not required. A separate data structure can be used to assign additional metadata to each label, such as a user-changeable name, description, highlight color or opacity. The visualization of the volumetric image can then be modified such that each voxel's color and opacity are combined with the color and opacity of the assigned label. In essence, this allows applying highlight colors and changing the opacity of entire segments, such as organs or pathological regions.

To hide, or apply a highlight color to segmented areas in a 3D volume, a simple modification to a standard ray-marching process can be used. In standard ray marching, at every step along a ray through the 3D volume a transfer function is used to map the scalar raw data of each voxel to respective opacity and color values of the respective voxel.

In addition to checking the opacity and color of the current ray marching voxel location within the 3D volume, the same voxel location is checked in the respective 3D label mask. If any label is assigned to the current voxel location (voxel), the color and opacity specified for that label is taken into account when computing the final color of that voxel. It is to be noted that ray-marching is not the focus of this document. A person skilled in the art is able to render 3D volumes that have associated 3D label masks by using ray marching. Rather, the focus in this document is on the replication of label masks being edited by one user to connected users in a collaborative session.

For example, a user can use a motion-tracked controller to interact with the virtual environment (including the 3D volume(s)), for example, to grab, move and rotate the loaded dataset. To edit the segmentation of a dataset, the user can activate a sphere-shaped segmentation object (e.g., a sphere-shaped brush tool) and interactively draw into the label mask. The brush can be represented by the outlines of a sphere. If the user moves this brush tool into the 3D dataset (e.g., CT data) and presses a button on the controller, any voxel within the sphere of the brush tool is assigned to the label currently selected for the brush tool (set-label mode). Alternatively, in a subtraction mode (unset-label mode), any voxel within the sphere which is already assigned to the currently selected label is removed from that assignment.

Determining which voxels are inside the sphere at a given moment - and should therefore be included in the processing of a label mask modification command (e.g., assigning or clearing the currently selected label associated with the segmentation object) - is in principle a trivial mathematical problem. The distance of every voxel's center point to the sphere's center is computed and compared to the sphere's radius. If the distance is bigger than the radius, then the voxel can be considered outside of the sphere, otherwise the label is assigned to the voxels inside the sphere. However, when determining if a voxel is inside the sphere based on the segmentation input data generated by an editing user, different GPUs in the front-end devices of the other connected users can compute different results and thereby arrive at opposite conclusions. This is caused by deviations from the IEEE 754 standard (IEEE Standard for Floating-Point Arithmetic," in IEEE Std 754-2019 (Revision of IEEE 754-2008), vol., no., pp.1-84, 22 July 2019, doi: 10.1109/IEEESTD.2019.8766229) allowed by some graphics libraries. For example, GPU manufacturers are allowed to opt for a different handling of certain edge cases in floating point operations. These allowed deviations result in different results with regard to the voxels that are determined to be inside the sphere of the segmentation object. As a consequence, the various connected users may see different virtual environments at the voxel level when their GPUs try to replicate the virtual environment state which corresponds to the segmentation state in the front-end device of the editing user. This problem arises when replicating the state based on the respective segmentation input data in accordance with approach b). In particular for medical applications, even minor deviations in the rendered label masks are not acceptable.

The above problem is solved by embodiments as claimed by the independent claims: a computer-implemented method for replicating a three-dimensional label mask for a three-dimensional volume dataset in a network of computing devices, a computer program product with instructions configured to cause execution of said method when executing the computer program product on a computing device, and a computer system which can load and execute said computer program product to execute said method.

In one embodiment, a computer-implemented method is provided for replicating a three-dimensional label mask for a three-dimensional volume dataset in a network of computing devices.

It is assumed that there is already a collaborative session running on the network where multiple users are connected via their respective computing devices. When a new user joins the collaborative session via his/her computing device, this additional computing device is initialized and synchronized with the entire dataset of the current label mask and the three-dimensional volume dataset. Hereinafter, this additional computing device of the joining user is also referred to as the first computing device. The entire three-dimensional volume dataset includes a 3D volume which is already loaded by the computing devices of all earlier connected users. The entire current label mask includes the 3D label mask with all voxels being assigned to one or more respective labels as being set after the latest editing/segmentation activity by an already connected user. Thereby, the voxels of the current label mask are associated with the corresponding voxels of the 3D volume data set. For each voxel of the label mask a label index can be stored for the corresponding voxel of the 3D volume. Multiple label masks for multiple 3D volumes can be replicated independently from each other.

Once the first computing device has been initialized, it is ready to receive a series of segmentation input data generated by a second computing device. The received segmentation input data reflect drawing commands which lead to changes of the label mask in comparison to the current label mask on the first computing device. This received series of segmentation input data is generated while the user of the second computing device is editing the label mask. For editing the label mask, a sphere-shaped segmentation object is used by the second computing device to allow its user to interactively draw into the label mask. The segmentation input data is transmitted to all connected users (possibly via a host), and is therefore also received by the first computing device. The segmentation input data comprise current position data, size data (i.e., the radius of the sphere) and a label identifier associated with the sphere-shaped segmentation object. Typically, during an editing/segmentation activity, the series of segmentation input data includes a plurality of label mask editing commands which describe the changing position of the segmentation object during a segmentation activity while the radius and the label of the segmentation object remain constant. However, it is also possible that during a segmentation activity the user changes the associated label identifier (e.g., switching to a label index representing a different segmentation color), and/or changes the mode of the segmentation object (e.g., set-label mode or unset-label mode), and/or changes the radius of the sphere (to adjust the size of the segmentation object).

The first computing device then replicates the edited label mask of the second computing device based on the received series of segmentation input data. Thereby, the first computing device performs the following steps for each segmentation input data of the received series:

Firstly, a respective ellipsoid is computed which corresponds to the sphere-shaped segmentation object stretched inverse proportionally to the shape of voxels in the label mask. Typically, the voxels of a volume have a cuboid shape (but not a cubic shape). In particular, voxels of volumes which are generated by medical imaging techniques, such as computer tomography imaging, magnetic resonance imaging, optical coherence tomography imaging, positron emission tomography imaging, single-photon emission computed tomography, and 3D ultrasound imaging. In such techniques, a plurality of two-dimensional scan images is composed into a three-dimensional volume. However, typically the distance between two 2D scan images is different from the edge size of the 2D pixels within a 2D scan image. This leads to a cuboid shape of the voxels. The computed ellipsoid has the purpose to adopt the sphere-shaped segmentation object to the cuboid voxel shape.

In a next step, the first computing device determines voxels of the label mask having a position inside the segmentation object. For this purpose, it computes the ratios of the distances between a respective voxel position and the center position of the ellipsoid in all three dimensions (referred to as numerators) divided by the respective semi-axis length of the ellipsoid (referred to as denominators). Thereby, the divisions are executed using bitwise operations on a graphical processing unit of the first computing device. Each semi-axis length is therefore a floating-point number represented by integer numbers for sign, significant and exponent. The divisions are executed by approximating the multiplicative inverse of the respective denominators by negating the exponents of the respective semi-axis lengths and multiplying the approximated multiplicative inverse of the respective denominators with their respective numerators. Using such bitwise operations ensures deterministic determination of the voxels inside the segmentation object. On the contrary, using the standard division instruction of the GPU leads to a label mask in the first computing device which deviates from the label mask generated by the second computing device in case GPUs of different types (e.g., GPUs from different manufacturers) are used by the two devices. Of course, the bitwise operations also work for such cases where the GPUs of the first and second computing devices are identical. In this case, the claimed method provides an alternative implementation for the implementation using the GPU standard division algorithm. It is to be noted that the implementation using bitwise operations may lead to a slightly different result than the normal division implementation resulting in a minor difference in the effective sphere size. However, this is irrelevant with regard to the requirement that all connected users need to see the same label mask which is always guaranteed by the bitwise operations allowing a deterministic computation which works for any GPU.

Finally, the first computing device updates the determined voxels in the replicated label mask in accordance with the label identifier of the segmentation input data. That is, after all pending segmentation input data have been processed (i.e., after completion of the updating step), the label mask of the first computing device is identical with the current label mask of the second computing device in all cases - independent of the type of GPU being used by the computing devices. That is, after replication of the label mask, the users of the respective devices see images rendered using the same segmentation information. For example, all voxels which were labeled with a particular color by the editing device are also rendered in the particular color on the replicating device. In collaborative sessions running within medical applications this is a critical requirement. Often, in such collaborative scenarios, different users use different VR devices and different GPUs from different manufactures. The herein proposed solution based on the bitwise division operations provides an accurate replication of the current label mask on each connected device.

To explain the bitwise division operations in more detail, a particular implementation of a division is described in the following. A division A/B can be expressed by multiplying A with an approximation of $B^{-1}$ being the inverse of B, wherein said approximation is computed by subtracting B, interpreted as an integer, from a predetermined integer constant. In accordance with the IEEE 754 standard for floating-point arithmetic, the exponent of a 32-bit float is stored in 8 bits following the sign bit. Because the exponent can be negative too, it is implemented using a bias of 128-1 = 127. That means, '127' is added to the actual exponent to keep it always positive.

The compiler of the first computing device is instructed to interpret the 32-bit float number as an integer number without changing the binary representation. This can be achieved by using programming-language-specific instructions, such as "floatBitsToUint" or "floatBitsToInt" in OpenGL Shading Language (GLSL), "asuint" or "asint" in High-Level Shading Language (HLSL), or by dereferencing an integer pointer which is set to the address of the float number in languages with explicit pointer support. The resulting integer is subtracted from an integer constant which is predetermined such that the result of said integer subtraction approximates the inverse of the float number by leveraging the standardized encoding format of floats, in particular the location of the exponent and the fraction in the binary representation. The result of the subtraction is reinterpreted as a float number resulting in an approximation of the inverse of the original float number.

For example, the interpreted integer may be subtracted from the hexadecimal number '0x7F000000' (the equivalent to the integer number '127' shifted to the 8 bits used for the exponent in a 32-bit float) as predetermined integer constant. Using '0x7F000000' as predetermined integer constant allows to implement a bitwise division operation where 1/1 exactly equals '1'. The bitwise division operation may also use other predetermined integer constants, such as for example '0x7EEEEEEE', which also leads to very good overall accuracy.

In particular in the context of medical applications it is important to proof the identity of the original label mask (second computing device) with the replicated label mask (first computing device). For this purpose, in an optional embodiment, the first computing device periodically receives, in addition to the segmentation input data, a hash value of the corresponding edited label mask of the second computing device. The first computing device then computes a hash value for the respective replicated label mask. If the two values are the same, the correctness of the replication of the label masks across the computing devices has been verified.

In further optional embodiments, the first computing device (and also the second computing device) may support a variety of updating modes. Besides the already mentioned 'set-label mode' to set the determined voxels in the replicated label mask to the label identifier of the segmentation input data, and the 'unset-label mode' to remove a particular earlier set label identifier from the determined voxels in the replicated label mask, any of the following update modes may be supported, too: 'clear-label' to reset all label identifiers of the determined voxels in the replicated label mask, 'set-label-for-unassigned-voxels' to set the label identifier of the segmentation input data in the replicated label mask only for determined voxels which are not yet labeled, and 'set-label-for-visible-voxels' to set the label identifier of the segmentation input data in the replicated label mask only for determined voxels which are visible in accordance with an applied transfer function. A person skilled in the art may define further update modes supported by the connected computing devices.

Optional embodiments may support label identifiers in the label mask of different sizes. For example, the label identifiers may be stored as 8-bit, 16-bit, 32-bit values, etc. for each voxel. For example, a label mask with 8-bit integer values for all voxels allows the definition of up to $2^8-1=255$ different labels. With the 32-bit embodiment this can be extended up to $2^{32}-1=4294967295$ labels. In an alternative embodiment, the value stored for each voxel in the label mask can be considered a bit field, where each bit corresponds to a different label. In this embodiment, the label mask can be used to assign a plurality of different labels to each voxel of the volumetric image. The number of different labels which can be simultaneously assigned to a voxel corresponds to the number of bits used for storing the values of the label mask.

In one embodiment, a computer program product is provided for replicating a three-dimensional label mask for a three-dimensional volume dataset in a network of computing devices. The computer program product comprises computer readable instructions that, when loaded into a memory of a computing device and executed by at least one processor of the computing device, cause the computing device to perform the computer-implemented method as disclosed herein.

In one embodiment, a computing device is provided for replicating a three-dimensional label mask for a three-dimensional volume dataset in a network of computing devices. The computing device implements functional software modules which are configured such that the computing device can perform the herein disclosed functions for executing said computer-implemented method.

In particular, the device has an interface adapted to receive, from the network of computing devices, a dataset of the current label mask and the entire three-dimensional volume dataset when joining a collaborative session on the network for initializing the computing device. A collaborative session as used herein is an interactive session involving multiple users with their respective computing devices, wherein the users are working toward a shared goal in real-time. The interface is further adapted to receive a series of segmentation input data from a further computing device of the network. The series of segmentation input data (editing commands) describes an editing of the label mask on the further computing device. Thereby, particular segmentation input data comprises current position data, size data and a label identifier associated with a sphere-shaped segmentation object used by the second computing device for interactively editing the label mask.

The system further has a replicator module adapted to replicate the edited label mask of the further computing device associated with the series of segmentation input data by processing each segmentation input data of the series with the following modules:

- an ellipsoid module adapted to compute a respective ellipsoid which corresponds to the sphere-shaped segmentation object stretched inverse proportionally to the shape of voxels in the label mask;
- a voxel identifier module adapted to determine voxels of the label mask having a position inside the segmentation object based on the ratios of the distances between a respective voxel position and the center position of the ellipsoid in all three dimensions divided by the respective semi-axis length of the ellipsoid. The divisions are implemented using bitwise operations on a graphical processing unit of the computing device to negate the exponent of the respective denominators; and
- an updating module adapted to update, in the replicated label mask, the determined voxels in accordance with the label identifier of the segmentation input data.

Advantageously, the voxel identifier implements the bitwise division operations for a division A/B by multiplying A with an approximation of $B^{-1}$ being the inverse of B, wherein said approximation is computed by subtracting B, interpreted as an integer, from a predetermined integer constant. This integer constant is predetermined such that the result of said integer subtraction approximates the inverse of the float number by leveraging the standardized encoding format of floats, in particular the location of the exponent and the fraction in the binary representation. The compiler of the computing device is instructed to interpret the 32-bit float number as an integer number without changing the binary representation. Then, the interpreted integer is subtracted from predetermined integer constant, e.g., the hexadecimal number '0x7F000000', the hexadecimal number '0x7FEEEEEE' (or another hex-number which complies with the above predetermination criterion). Finally, the result of the subtraction is reinterpreted as a float number resulting in an approximation of the inverse of the original float number with a negated exponent.

Further aspects of the invention will be realized and attained by means of the elements and combinations particularly depicted in the appended claims. It is to be understood that both the foregoing general description and the following detailed description are exemplary and explanatory only, and are not restrictive of the invention as described.

**Brief Description of the Drawings**

FIG. 1 illustrates a block diagram including an embodiment of a computing device for replicating a three-dimensional label mask for a three-dimensional volume dataset in a network of computing devices;

FIG. 2A is a simplified flow chart illustrating a computer-implemented method for replicating a three-dimensional label mask for a three-dimensional volume dataset in a network of computing devices according to an embodiment;

FIG. 2B is a simplified flow chart illustrating a bit-wise operation example for executing a division on a GPU to determine voxels inside a segmentation object;

FIGs. 3A to 3B are screenshots illustrating an example of an editing activity modifying the segmentation of a three-dimensional object with a segmentation object;

FIG. 4A illustrates voxels of a 3D volume and its coordinate system;

FIG. 4B illustrates the typical stretched, cuboid shape of voxels of a volumetric dataset and the spherical shape of the segmentation tool as seen by the user;

FIG. 4C illustrates the same volumetric dataset and segmentation tool transformed such that the voxels become cuboid shaped;

FIG. 5 is a representation of a 32-bit real number;

FIGs. 6A, 6B illustrate simplified 2D views of a segmentation result and a replicated segmentation result on two different GPUs;

FIG. 7 illustrates an example segmentation result in a medical application with segmentation applied to the spine of a human skeleton;

FIG. 8 shows a photo of a collaborative session with four users and four connected computing devices.

**Detailed Description**

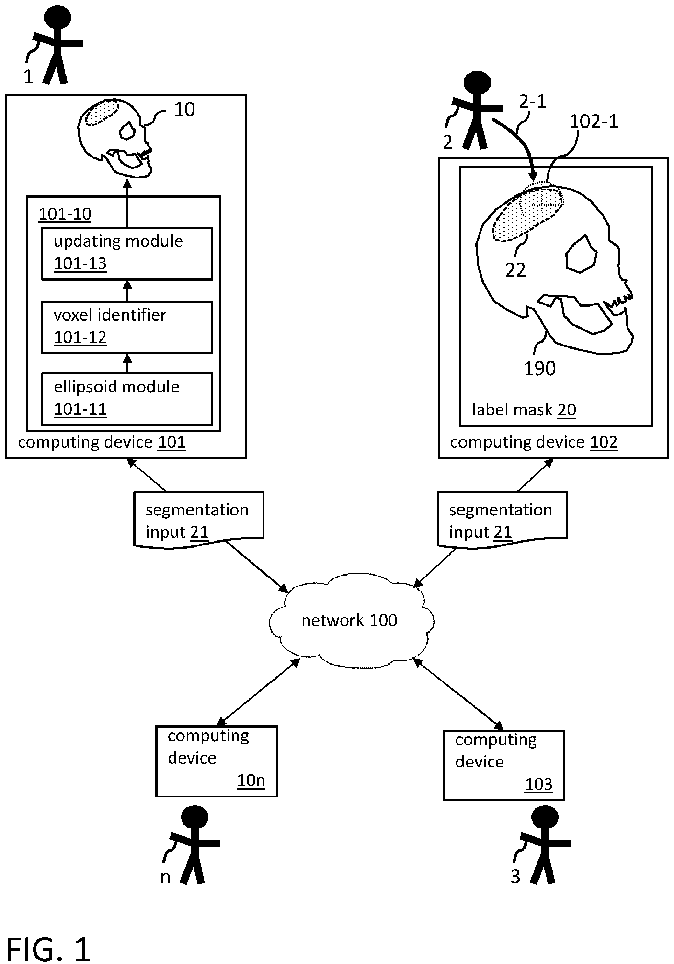

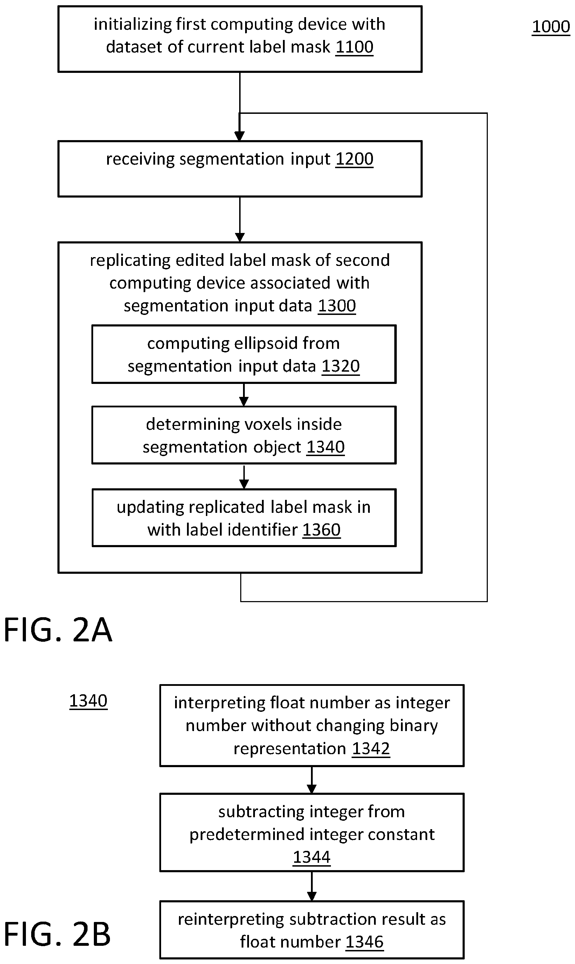

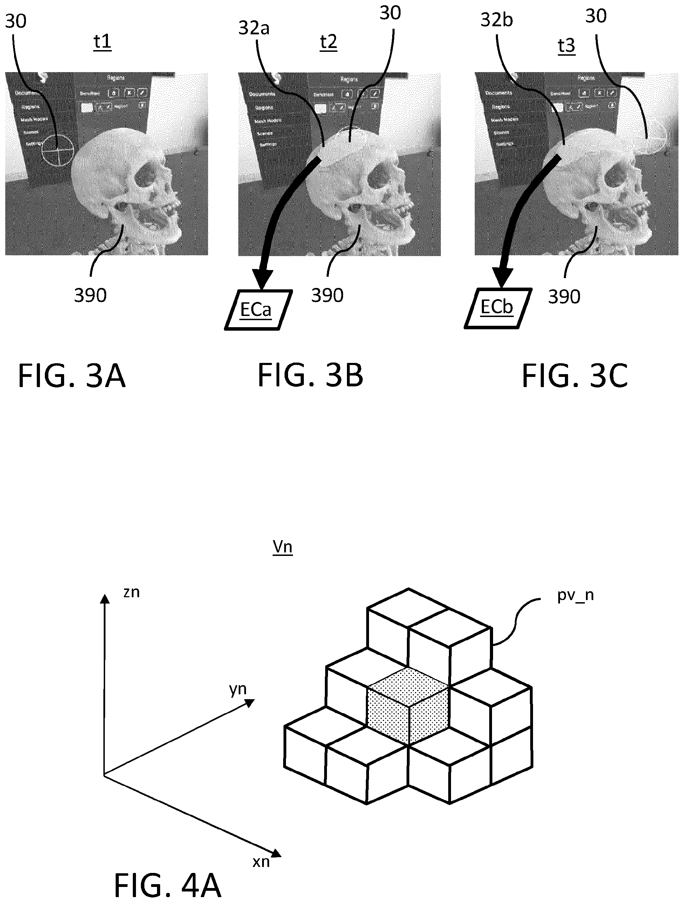

FIG. 1 illustrates a block diagram including an embodiment of a computing device 101 for replicating a three-dimensional label mask 20 for a three-dimensional volume dataset 190 in a network 100 of computing devices 101 to 10n (the computing devices communicating via the network). In the example of FIG. 1, only the visible voxels of the volume data set 190 are illustrated. The entire 3D volume data set however has a cuboid shape. FIG. 2A is a simplified flow chart illustrating a computer-implemented method 1000 for replicating the three-dimensional label mask for the three-dimensional volume dataset in a network of computing devices according to an embodiment. For example, method 1000 can be executed by computing device 101. Therefore, FIG. 1 is described now in the context of FIG. 2. For this reason, reference numbers of FIG. 1 and FIG. 2 are used in the following description. Further, FIGs. 3A to 3B are screenshots illustrating an example of an editing activity modifying the segmentation 32a, 32b of a three-dimensional object (three-dimensional volume dataset 390) with a segmentation object 30. This example will also be used in the context of the description of FIGs. 1 and 2A.

The computing device 101 is connected with other computing devices 102, 103, ..., 10n over the network 100 for running a collaborative session (by executing a collaborative application). The computing devices have standard communication interfaces allowing the exchange of data between the devices. Each computing device is operated by a respective user 1, 2, 3, ..., n. In the following, an example is described where computing device 101 receives data from computing device 102 for replicating the label mask 20. It is to be noted that in the described example scenario, computing devices 103 to 10n can perform the same functions as described for computing device 101 to replicate the label mask 20 of computing device 102 using their respective GPUs. Further, one or more of the computing devices 101, 103 to 10n may also be used as editing devices for their own respective label masks in which case all the other connected devices can replicate the corresponding modified label masks.

In the collaborative example session of FIG. 1, computing device 102 is running an application which allows editing 2-1 of a three-dimensional volume dataset 190. In the example, the application is a medical application which renders a 3D volume with CT scan of a human skull. However, any other application for visualizing 3D objects to a user can make use of the herein described approach. A volume consists of so-called voxels. FIG. 4A illustrates some voxels pv_n of a volume Vn. Voxels of a volume have a cuboid shape. In the complete volume Vn, each voxel layer has the same number of voxels arranged in a rectangular shape leading to a cuboid shape of the volume. The voxels may have a cubic shape, but in general, the volume Vn is typically composed from layers of 2D images where each pixel of a 2D image is rectangular shaped. In the example, it is assumed that the 2D image layers are located in the xn-yn plane of the local coordinate system CSn of Vn. When looking at the dotted voxel it can be seen that the depth of the voxel in the zn dimension is different from the side (edge) lengths of the voxel in the xn-zn dimensions. The distance at which the 2D image layers are captured (e.g., by any of the earlier mentioned volumetric imaging techniques) corresponds to the depth of the voxels in the zn-dimension. Typically, the depth of a voxel is different from the edge lengths of the respective pixel in the underlying 2D image layers.

Turning back to the example of FIG. 1, user 2 uses a sphere-shaped segmentation object 102-1 to interactively draw into the label mask 20 associated with the 3D volume dataset 190. Thereby, the segmentation object is used as a brush tool which the user can use to interactively draw into the 3D dataset. Voxels which are touched by the brush tool are labeled accordingly. The label mask 20 is also a 3D volume dataset which can store label values for each voxel of the 3D dataset 190. The segmentation area 22 indicates such voxels of the label mask which were touched by the brush tool 102-1 during editing 2-1. The labeled voxels (e.g., having a color label which was set for the segmentation object) are visualized for the user 2 in such a way that the labeled voxels can be clearly distinguished from non-labeled voxels.

The editing commands generated while user 2 is editing 2-1 the label mask 20 are also referred to as segmentation input data 21 herein. During the editing 2-1 activity, typically a series of segmentation input data is generated while the segmentation object 102-1 is being moved. Particular segmentation input data comprises current position data, size data and a label identifier associated with the sphere-shaped segmentation object. The example in FIGs. 3A to 3C illustrates editing the label mask associated with 3D volume dataset (skull) 390 at three different time points t1, t2, t3. At t1 (cf. FIG. 3A), the user approaches skull 390 with the segmentation object 30 but the segmentation object does not yet intersect with the skull. At t2 (cf. FIG. 3B), the segmentation object has already labeled a plurality of voxels 32a of the label mask. While the segmentation object 30 intersects with the skull 390, the system records corresponding editing command ECa as segmentation input data. That is, ECa stands for a series of segmentation input data which reflect respective position data, size data and label identifier during the editing movement. At t2, the segmentation object is "penetrating" the skull and the right portion of the sphere surface intersecting with the skull defines the current edge of the labeled voxel area 32a. At t3 (cf. FIG. 3C), the segmentation activity is finalized and the user has moved the segmentation object 30 away from the skull. The area 32b indicating the voxels that had been labeled during the editing movement is now larger than at t2. That is, a further series of editing commands ECb was applied to the label mask between t3 and t2.

Turning back to FIG. 1, in the collaborative session over network 100, the current label mask 20 with the labeled voxels 22 on computing device 102 needs to be replicated to the connected computing devices in real-time. In the following, the replication of the label mask is only described for computing device 101. However, any of the other connected devices can implement the same replication method as computing device 101.

When computing device 101 joins the collaborative session on the network 100 for the first time, an initializing step 1100 is performed where the computing device 101 initially receives (via its communication interface) from the collaborative session's host the current label mask of the collaborative session and the entire three-dimensional volume dataset 190. The collaborative session host receives all editing commands of all connected devices thus knowing always the exact current label mask (because it knows the true sequential order of all editing commands). The current label mask can then be provided to any replicating device. The collaborative session host can be seen as an intermediary for forwarding editing commands from an editing device to a replicating device while ensuring that all editing commands originating in different editing devices are always being processed in the right order. Once computing device 101 is initialized it can replicate changes to the label mask applied by any of the connected devices. In the example, computing device 102 has performed the latest editing action on the label mask 20. The respective series of segmentation input data (editing commands) is provided to the network 100 by computing device 102 and received 1200 by computing device 101. As defined earlier, the series of segmentation input data 21 describes the editing 2-1 of the label mask 20 by the editing commands generated on computing device 102. That is, after having received the segmentation input data 21, computing device 101 knows the positions of the segmentation object 102-1, its size and the associated label which needs to be assigned to such voxels of the label mask which intersected with the sphere-shaped segmentation object during the editing activity on computing device 102.

Computing device 101 has a replicator module 101-10 adapted to replicate 1300 the edited label mask of computing device 102 associated with the series of segmentation input data 21 by processing each segmentation input data of the series with its following (sub-) modules:

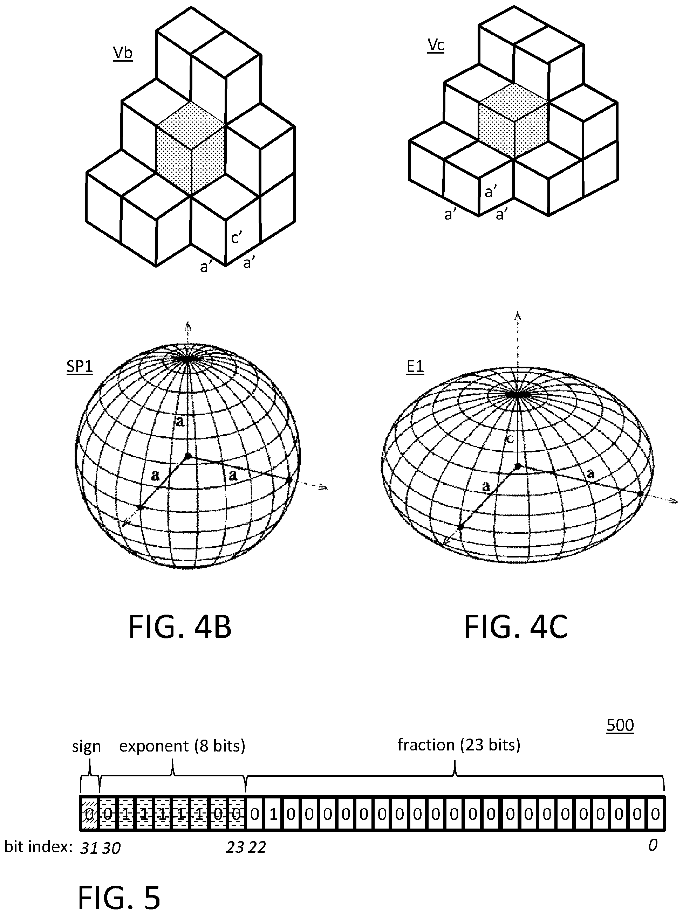

An ellipsoid module 101-11 computes 1320 a respective ellipsoid which corresponds to the sphere-shaped segmentation object 102-1 stretched inverse proportionally to the shape of voxels in the label mask. FIGs. 4B and 4C illustrate such inverse proportional stretching of the sphere-shaped segmentation object into a corresponding ellipsoid. For checking whether a voxel is inside the sphere of SP1, the distance D of a voxel at position $(v_x, v_y, v_z)$, whereby the voxel position is defined at the center of the voxel, to the center $(c_x, c_y, c_z)$ of the sphere is given by formula F1:

$$D = \sqrt{(v_x - c_x)^2 + (v_y - c_y)^2 + (v_z - c_z)^2}$$

To simplify, the square root can be removed, and the result can be directly compared to the value to the squared radius $a^2$ of the sphere SP1.

However, as mentioned earlier, in real volumes typically the size of the voxels in the three dimensions is not uniform. That is, the edge length of a voxel cuboid reflecting the distance between the two image layers in a 3D volume is different from the edge length defining the pixel size in a single layer. For example, in CT images, the CT slice distance (z-axis) is usually bigger, than the pixel size in each slice. A typical voxel size in CT scans would be 0.3mm x 0.3mm x 1.5mm.

FIG. 4B illustrates such a situation where the edge length of the pixels in a layer (slice) is $a'$ but the edge length between two layers (lice distance) is $c' > a'$, thus resulting in a cuboid voxel shape of the voxels of volume Vb. When the sphere-shaped segmentation object SP1 now intersects with the voxels of volume Vb, less voxels fit into the c'-dimension than into the a'-dimension. For the following computations, it is advantageous to operate on cubic voxels because cuboid voxels cannot be represented in a texture (whether a 2D image or a 3D volume) on a GPU. Such textures are always simply a uniform grid of 2D or 3D values. This is illustrated as the volume Vc in FIG. 4C. Therefore, a segmentation object that appears in the shape of a sphere to the user, needs to be treated as a sphere that is stretched inverse proportionally to the shape of voxels, i.e., an ellipsoid. This can be achieved by using the inverse proportionally stretched ellipsoid E1 where the c-axis length is inversely proportionally reduced in comparison to the semi-axis c.

To determine whether a voxel at position $(v_x, v_y, v_z)$ is inside the spherical segmentation object, formula F2 for an ellipsoid at center position $(c_x, c_y, c_z)$ and semi-axes length $(a_x, a_y, a_z)$ can be used:

$$\left(\frac{v_x - c_x}{a_x}\right)^2 + \left(\frac{v_y - c_y}{a_y}\right)^2 + \left(\frac{v_z - c_z}{a_z}\right)^2 < 1$$

Therefore, a voxel identifier module 101-12 of computing device 101 is adapted to determine 1340 voxels of the label mask having a position inside the segmentation object based on the ratios of the distances between a respective voxel position and the center position of the ellipsoid in all three dimensions divided by the respective semi-axis length of the ellipsoid. An issue arises when implementing such divisions on a GPU, because the position and radius of the sphere can be any real number, and therefore the system has to work with floating-point numbers. But even with integer values, the divisions will likely produce fractional numbers. Floating-point numbers (floats) in CPU and GPU memory are represented by three integer numbers: the sign, the significand, and the exponent.

Floating-point numbers (floats) in CPU and GPU memory are represented by 3 integer numbers: the sign, the significand, and the exponent. FIG. 5 illustrates an example of a single precision (32-bit) float 500 as defined by the IEEE 754 standard for floating-point arithmetic. The 23 bits long fraction portion of float 500 is stored in bits '0' to '22'. The 8 bits long exponent portion is in bits '23' to '30' and the sign bit is stored in bit 31. The float number value represented by the 32-bit float 500 is: '0.15625'. A problem arises from the fact that the precision of a float is limited, and therefore, cannot represent every possible real number in 32 bits. As a consequence, some results of the above-mentioned divisions of F2 will be real numbers that cannot be represented accurately as a float.

IEEE 754 requires correct rounding: that is, the rounded result is as if infinitely precise arithmetic was used to compute the value and then rounded to the nearest value which can be represented as a float. Alternative rounding modes are specified by IEEE 754 in section 4.3. On GPUs, certain deviations from the standard are allowed by some graphics libraries, such as Microsoft's DirectX. This is mainly due to the fact that GPUs are mainly used for video games and visual effects, where minor inaccuracies are not an issue and determinism is rarely required. Critically, GPU manufacturers are allowed to opt for non-standard rounding modes and lower precision results for operations, such as the square root or divisions (cf. White, S., Coulter, D., Jacobs, M., Satran, M., "Floating-point rules (Direct3D 11)", Programming Guide for Direct3D 11, Microsoft, 2020, Accessed 23.11.2022, https://learn.microsoft.com/en-us/windows/win32/direct3d11/floating-point-rules): "IEEE-754 requires floating-point operations to produce a result that is the nearest representable value to an infinitely-precise result, known as round-to-nearest-even. Direct3D 11 defines the same requirement: 32-bit floating-point operations produce a result that is within 0.5 unit-last-place (ULP) of the infinitely-precise result. This means that, for example, hardware is allowed to truncate results to 32-bit rather than perform round-to-nearest-even, as that would result in error of at most 0.5 ULP. This rule applies only to addition, subtraction, and multiplication.

sqrt and rcp have 1 ULP tolerance. The shader reciprocal and reciprocal square-root instructions, rcp and rsq, have their own separate relaxed precision requirement.

Multiply and divide each operate at the 32-bit floating-point precision level (accuracy to 0.5 ULP for multiply, 1.0 ULP for reciprocal). If x/y is implemented directly, results must be of greater or equal accuracy than a two-step method."

As a result of these allowed rounding deviations, if two connected computing devices use GPUs from different manufacturers, the devices may arrive at different label mask replication results despite starting with the same initial state and executing the same label mask drawing command (i.e., the same segmentation input data). Theoretically, even different GPU models or series from the same manufacturer, or the exact same GPU after a graphics driver update, may change the behavior in the above-mentioned situations, since the decision is left up to the manufacturer.

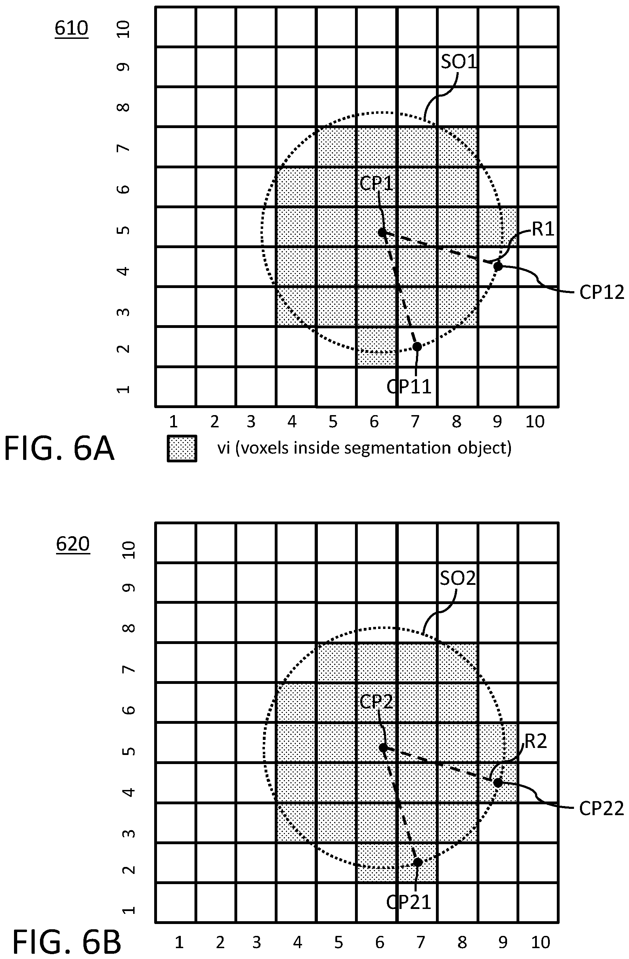

This problem is visualized in FIGs. 6A, 6B. For simplification, only 2D projections of the segmentation object and respective voxels are shown. FIG. 6A is supposed to show the label mask 610 which is edited by a user on the respective computing device. It is assumed that the label mask was empty (no voxels labeled) before the editing command of the user. The user has positioned the segmentation object SO1 such that the center CP1 of SO1 is inside of the voxel at position (6, 5) of the label mask. Typically, the center CP1 does not coincide with the center of a voxel. The radius R1 of SO1 is illustrated by dashed lines. For example, the GPU of the editing device may follow IEEE 754 and always round the result of a floating-point operation to the nearest representable float. The voxels which are determined according to F2 as being located inside SO1 (that is, the center of the respective voxel is inside SO1) have a dotted texture. In the example, the voxels at positions (7, 2) and (9, 4) have their center points CP11, CP12 close to the surface of 501, but such that the GPU of the editing device comes to the result that they are not inside 501.

FIG. 6B illustrates the label mask 620 as generated by the replicating computing device with a GPU using a different rounding method. For example, the GPU of the editing device may follow IEEE 754 and always round the result of a floating-point operation to the nearest representable float. The GPU of the replicating device may opt for the allowed shortcut of truncating the result (always rounding down). The sphere SO2 is reproduced based on the segmentation input data in the editing computing device: position CP1 = CP2 and radius R1 = R2. However, the GPU of the replicating device comes to the result that the voxels at positions (7, 2) and (9, 4) have their center points CP21, CP22 inside the replicated sphere SO2 because the GPU of the replicating device computes a slightly different distance to the center of the sphere for a given voxel than the editing device. Therefore, these voxels are also labeled with the label indicated in the segmentation input data (illustrated by dotted texture). The users of the editing device and the replicating device would therefore see some differences in the label assignments in the label mask. In particular in medical applications, such deviations in the label mask of the replicating device which is supposed to reflect the segmentation result on the editing device is not acceptable.

Optionally, for detecting such differences between the label masks of different computing devices, a hash of the entire label mask may be computed on each computing device and may be compared with the hash value on the computing device of the collaborative session host. If the hash values are different, the host of the session may transmit the authoritative version of the label mask to the connected computing devices of users with an out-of-sync hash value. However, this process takes a few seconds and can lead to data loss and should preferably be avoided in a real-time collaborative session.

To avoid such disadvantages, the herein disclosed approach provides method and systems which avoid the generation of different label masks on the replicating computing devices independent of the GPUs being used. This is achieved by implementing the divisions of formula F2 to determine whether a voxel is inside an ellipsoid or not by using bitwise operations on the GPUs of the connected computing devices (in the example of FIG. 1, in particular the editing device 102 and the replicating device 101) to negate the exponent of the respective denominators. This can be done because of formula F3:

$$\frac{a}{b} = a\frac{1}{b} = a \times b^{-1}$$

That is, the claimed approach does not use the GPU's floating-pointing division. The result of the bit-wise operations is deterministic and reproducible on any GPU.

Finally, an updating module 101-13 of the replicating computing device 101 is adapted to update 1360, in the replicated label mask 10 (i.e., the label mask being generated by the replicating device 101 in response to the received segmentation input data 21), the determined voxels in accordance with the label identifier of the received segmentation input data 21.

FIG. 2B illustrates sub-steps of the determining step 1340 which show details of an example implementation of the bit-wise division operations for a division A/B by multiplying A with an approximation of $B^{-1}$ being the inverse of B, in accordance with formula F3, wherein the exponent is stored in 8 bits following the sign bit of a 32-bit float (cf. FIG. 5) using a bias of '127'. In this implementation, the compiler of the computing device is instructed to interpret 1342 the 32-bit float number as an integer number without changing the binary representation. In the example implementation, the interpreted integer is subtracted 1344 from the hexadecimal number 0x7F000000 (corresponding to integer value '127' shifted to the location of the 8 bits storing the exponent), and the result of the subtraction is reinterpreted 1346 as a float number resulting in an approximation of the inverse of the original float number (in this implementation with a negated exponent).

Turning back to FIG. 1, the segmentation input data 21 may further include an update mode associated with the segmentation object while editing 2-1 the label mask 20 on computing device 102 (i.e., the editing device). When using the spherical segmentation object, user 2 can select a segmentation mode which determines how the voxels falling inside the spherical segmentation object are to labeled. From the perspective of the replicating devices, the segmentation mode is applied when updating the corresponding replicated label masks. For this reason, the segmentation mode is also referred to as update mode when being received as part of the segmentation input data. That is, the selected segmentation (update) mode becomes then part of the segmentation input data 21 provided by the editing device 102 to the connected devices. The following list illustrates examples of possible update modes (but the list is not meant to be limiting - a person skilled in the art may define further update modes):

- set-label mode: for setting the determined voxels in the replicated label mask to the label identifier of the segmentation input data

- unset-label mode: for removing a specific earlier set label identifier from the determined voxels in the replicated label mask

- clear-label mode: for resetting all label identifiers of the determined voxels in the replicated label mask (reset a label identifier means setting the label value to its initial value, e.g., to '0')

- set-label-for-unassigned-voxels mode: for setting the label identifier of the segmentation input data in the replicated label mask only for determined voxels which are not yet labeled

- set-label-for-visible-voxels mode: for setting the label identifier of the segmentation input data in the replicated label mask only for determined voxels which are visible in accordance with an applied transfer function.

The label identifiers in the label mask may typically be stored as 8-bit or 16-bit or 32-bit values for each voxel. Larger bit sizes are possible by using multiple texture channels and/or multiple textures. The label values may be interpreted in different ways. In one implementation, the bits of the label identifiers in the label mask are interpreted a single integer label value for each voxel. That is, the label values can cover a large value range but there can only be a single label for each voxel. In another implementation, each bit of the label identifier may be interpreted as a label. That is, there can be as many labels assigned to a voxel as the number of bits of the label identifier.

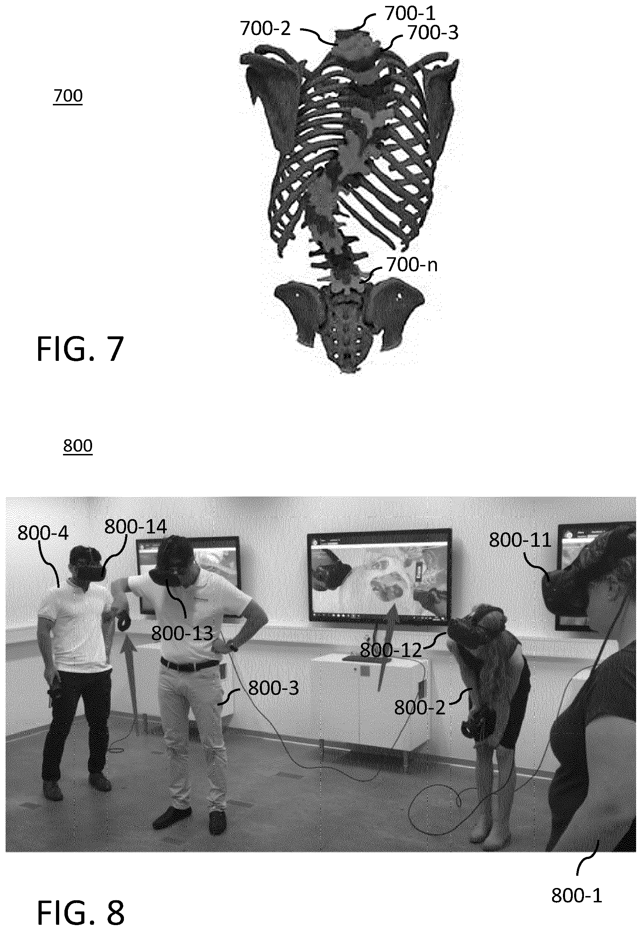

FIG. 7 illustrates an example segmentation result in a medical application with segmentation applied to the spine of a human skeleton 700. Each spine vertebra 700-1 to 700-n has been segmented with a distinguishing segmentation color such that neighbored vertebrae are assigned to different segmentation colors. That is, the respective voxels in the label mask have been assigned to the label indicator associated with the color value associated with the segmentation objection during the respective editing activity. When replicating the label mask based on the editing commands for the spherical segmentation object in accordance with the herein disclosed approach using bit-wise division operations, the exact label mask is reproduced at each of the connected replicating devices.

FIG. 8 shows a photo of a real-world collaborative session 800 with four users 800-1 to 800-4 and four connected computing devices 800-11 to 800-14 (virtual reality devices). In the example, the collaborative session is part of a neurosurgery course with a surgeon 800-3 and three students 800-1, 800-2 and 800-4 performing a virtual surgery in a shared virtual environment. The surgeon is removing part of a bone in a CT scan of a patient using a spherical segmentation object in an eraser mode. An eraser mode could be implemented by using the set-label mode of the segmentation object in combination with a label that has been assigned a completely transparent color value. This operation is then synchronized to the students' virtual reality devices over the network, so they see the changes (edits) applied by the surgeon to the label mask (from their own perspective).

**Claims**

1. A computer-implemented method (1000) for replicating a three-dimensional label mask (20) for a three-dimensional volume dataset (190) in a network (100) of computing devices (101 to 10n), comprising:

   upon a first computing device (101) joining a collaborative session on the network (100), initializing (1100) the first computing device (101) with the entire dataset of the current label mask and the three-dimensional volume dataset (190);
   receiving (1200), at the first computing device (101), a series of segmentation input data (21) generated by a second computing device (102), wherein particular segmentation input data comprises current position data, size data and a label identifier associated with a sphere-shaped segmentation object (102-1) used by the second computing device (102) for interactively editing the label mask;
   replicating (1300), at the first computing device (101), the edited label mask of the second computing device (102) associated with the series of segmentation input data by: for each segmentation input data of the series,

      computing (1320) a respective ellipsoid which corresponds to the sphere-shaped segmentation object (102-1) stretched inverse proportionally to the shape of voxels in the label mask;
      determining (1340) voxels (vi) of the label mask having a position inside the segmentation object based on the ratios of the distances between a respective voxel position and the center position of the ellipsoid in all three dimensions as numerators divided by the respective semi-axis length of the ellipsoid as denominators, each semi-axis length being a floating-point number represented by integer numbers for sign, significand and exponent,
      wherein the divisions are executed on a graphical processing unit of the first computing device (101) by approximating the multiplicative inverse of the respective denominators by negating the exponents of the respective semi-axis lengths using bitwise operations, and multiplying the approximated multiplicative inverse of the respective denominators with their respective numerators;
      and updating (1360), in the replicated label mask (10), the determined voxels in accordance with the label identifier of the segmentation input data (21).

2. The method of claim 1, wherein the bitwise division operations implement a division A/B by multiplying A with an approximation of $B^{-1}$ being the inverse of B, wherein said approximation is computed by subtracting B, interpreted as an integer, from a predetermined integer constant, in that:

   a compiler of the first computing device is instructed to interpret (1342) the 32-bit float number as an integer number without changing the binary representation,
   the resulting integer is subtracted (1344) from the predetermined integer constant being predetermined such that the result of said integer subtraction approximates the inverse of the float number by leveraging the standardized encoding format of floats, in particular the location of the exponent and the fraction in the binary representation, and
   the result of the subtraction is reinterpreted (1346) as a float number resulting in an approximation of the inverse of the original float number.

3. The method of claim 1 or 2, wherein the collaborative session is running within a medical application.

4. The method of any of the previous claims, wherein the first computing device (101) receives, together with the segmentation input data (21), a hash value of the corresponding edited label mask (20) of the second computing device (102), and further computes a hash value for the respective replicated label mask (10) to ensure correctness of the replication of the label masks across the computing devices.

5. The method of any of the previous claims, wherein updating is performed using at least a set-label mode to set the determined voxels in the replicated label mask to the label identifier of the segmentation input data, and an unset-label mode to remove a specific earlier set label identifier from the determined voxels in the replicated label mask.

6. The method of claim 5, wherein updating is performed using any of the following update modes: clear-label to reset all label identifiers of the determined voxels in the replicated label mask, set-label-for-unassigned-voxels to set the label identifier of the segmentation input data in the replicated label mask only for determined voxels which are not yet labeled, and set-label-for-visible-voxels to set the label identifier of the segmentation input data in the replicated label mask only for determined voxels which are visible in accordance with an applied transfer function.

7. The method of any of the previous claims, wherein, for each voxel of the label mask, the label identifier of a single label is stored as an 8-bit, 16-bit or 32-bit value.

8. The method of claim 7, wherein, for each voxel of the label mask, an 8-bit, 16-bit, 32-bit or longer bit field is stored, where each bit corresponds to a label, thereby allowing a plurality of labels to be set for each voxel.

9. A computer program product for replicating a three-dimensional label mask (20) for a three-dimensional volume dataset (190) in a network (100) of computing devices (101 to 10n), the computer program product comprising computer readable instructions that, when loaded into a memory of a computing device and executed by at least one processor of the computing device, cause the computing device to perform the method according to any one of the previous claims.

10. A computing device (101) for replicating a three-dimensional label mask (20) for a three-dimensional volume dataset (190) in a network (100) of computing devices, comprising:

an interface (110) adapted to receive, from the network of computing devices, a dataset of the current label mask and the entire three-dimensional volume dataset (190) when joining a collaborative session on the network (100) for initializing the computing device (101), and further adapted to receive a series of segmentation input data (21) generated by a further computing device (102) of the network, the series of segmentation input data describing an editing (2-1) of the label mask (20) on the further computing device (102), wherein particular segmentation input data comprises current position data, size data and a label identifier associated with a sphere-shaped segmentation object (102-1) used by the second computing device (102) for interactively editing the label mask;

a replicator module (101-10) adapted to replicating the edited label mask of the further computing device (102) associated with the series of segmentation input data by processing each segmentation input data of the series with the following modules:

an ellipsoid module (101-11) adapted to compute a respective ellipsoid which corresponds to the sphere-shaped segmentation object (102-1) stretched inverse proportionally to the shape of voxels in the label mask; a voxel identifier module (101-12) adapted to determine voxels (vi) of the label mask having a position inside the segmentation object based on the ratios of the distances between a respective voxel position and the center position of the ellipsoid in all three dimensions as numerators divided by the respective semi-axis length of the ellipsoid as denominators, each semi-axis length being a floating-point number represented by integer numbers for sign, significand and exponent, wherein the divisions are implemented on a graphical processing unit of the computing device (101) by approximating the multiplicative inverse of the respective denominators by negating the exponents of the respective semi-axis lengths using bitwise operations, and multiplying the approximated multiplicative inverse of the respective denominators with their respective numerators; and

an updating module (101-13) adapted to update, in the replicated label mask (10), the determined voxels in accordance with the label identifier of the segmentation input data (21).

11. The device of claim 10, wherein the voxel identifier implements the bitwise division operations for a division A/B by multiplying A with an approximation of $B^{-1}$ being the inverse of B, wherein said approximation is computed by subtracting B, interpreted as an integer, from a predetermined integer constant being predetermined such that the result of said integer subtraction approximates the inverse of the float number by leveraging the standardized encoding format of floats, in particular the location of the exponent and the fraction in the binary representation, in that:

a compiler of the computing device is instructed to interpret the 32-bit float number as an integer number without changing the binary representation, the resulting integer is subtracted from the predetermined integer constant, and the result of the subtraction is reinterpreted as a float number resulting in an approximation of the inverse of the original float number.

**12.** The device of claim 10 or 11, wherein the collaborative session is a session being executed within a medical application.

**13.** The device of any of the claims 10 to 12, wherein the computing device (101) is further adapted to receive, together with the segmentation input data (21), a hash value of the corresponding edited label mask (20) of the further computing device (102), and to compute a hash value for the respective replicated label mask (10) to ensure correctness of the replication of the label masks across the two computing devices.

**14.** The device of any of the claims 10 to 13, wherein the updating module is further adapted to support any one of a plurality of update modes comprising: a set-label mode to set the determined voxels in the replicated label mask to the label identifier of the segmentation input data, an unset-label mode to remove a specific earlier set label identifier from the determined voxels in the replicated label mask, clear-label mode to reset all label identifiers of the determined voxels in the replicated label mask, a set-label-for-unassigned-voxels mode to set the label identifier of the segmentation input data in the replicated label mask only for determined voxels which are not yet labeled, and a set-label-for-visible-voxels mode to set the label identifier of the segmentation input data in the replicated label mask only for determined voxels which are visible in accordance with an applied transfer function.

**15.** The device of any of the claims 10 to 14, wherein the bits of the voxel values in the label mask represent a single integer label value for each voxel, or wherein each bit of the voxel values in the label mask corresponds to a separate label for the corresponding voxel.

**Patentansprüche**

**1.** Computerimplementiertes Verfahren (1000) zum Replizieren einer dreidimensionalen Etikettenmaske (20) für einen dreidimensionalen Volumendatensatz (190) in einem Netzwerk (100) von Rechenvorrichtungen (101 bis 10n), umfassend:

wenn eine erste Rechenvorrichtung (101) einer kollaborativen Sitzung im Netzwerk (100) beitritt, Initialisieren (1100) der ersten Rechenvorrichtung (101) mit dem gesamten Datensatz der aktuellen Etikettenmaske und dem dreidimensionalen Volumendatensatz (190);
Empfangen (1200), an der ersten Computervorrichtung (101), einer Reihe von Segmentierungseingabedaten (21), die von einer zweiten Computervorrichtung (102) erzeugt werden, wobei bestimmte Segmentierungseingabedaten aktuelle Positionsdaten, Größendaten und einen Etiketten-Identifikator umfassen, der einem kugelförmigen Segmentierungsobjekt (102-1) zugeordnet ist, das von der zweiten Computervorrichtung (102) zum interaktiven Bearbeiten der Etikettenmaske verwendet wird;
Replizieren (1300), an der ersten Rechenvorrichtung (101), der bearbeiteten Etikettenmaske der zweiten Rechenvorrichtung (102), die der Serie von Segmentierungseingabedaten zugeordnet ist, durch: für alle Segmentierungseingabedaten der Serie,

Berechnen (1320) eines jeweiligen Ellipsoids, das dem kugelförmigen Segmentierungsobjekt (102-1) entspricht, das umgekehrt proportional zur Form der Voxel in der Etikettenmaske gestreckt ist;
Bestimmen (1340) von Voxeln (vi) der Etikettenmaske, die eine Position innerhalb des Segmentierungsobjekts haben, basierend auf den Verhältnissen der Abstände zwischen einer jeweiligen Voxelposition und der Mittelpunktsposition des Ellipsoids in allen drei Dimensionen als Nenner, dividiert durch die jeweilige Halbachsenlänge des Ellipsoids, wobei jede Halbachsenlänge eine Gleitkommazahl ist, die durch ganze Zahlen für Vorzeichen, Mantisse und Exponent dargestellt wird, wobei die Divisionen auf einer grafischen Verarbeitungseinheit der ersten Rechenvorrichtung (101) ausgeführt werden, indem der Kehrwert der jeweiligen Nenner durch Negieren der Exponenten der jeweiligen Halbachsenlängen unter Verwendung bitweiser Operationen angenähert wird und der angenäherte Kehrwert der jeweiligen Nenner mit ihren jeweiligen Zählern multipliziert wird;
und Aktualisieren (1360) der bestimmten Voxel in der replizierten Etikettenmaske (10) gemäß dem Etiketten-Identifikator der Segmentierungseingangsdaten (21).

**2.** Verfahren nach Anspruch 1, wobei die bitweisen Divisionsoperationen eine Division A/B durch Multiplizieren von A mit einer Annäherung von $B^{-1}$, das der Kehrwert von B ist, implementieren, wobei die Annäherung durch Subtrahieren von B, das als eine ganze Zahl interpretiert wird, von einer vorbestimmten ganzzahligen Konstante berechnet wird, indem:

ein Compiler der ersten Rechenvorrichtung angewiesen wird, die 32-Bit-Gleitkommazahl als ganze Zahl zu interpretieren (1342), ohne die binäre Darstellung zu ändern,

die sich ergebende ganze Zahl von der vorbestimmten ganzzahligen Konstante subtrahiert wird (1344), die so vorbestimmt ist, dass das Ergebnis der ganzzahligen Subtraktion sich dem Kehrwert der Gleitkommazahl annähert, indem das standardisierte Kodierungsformat von Gleitkommazahlen, insbesondere die Position des Exponenten und des Bruches in der binären Darstellung, genutzt wird, und

das Ergebnis der Subtraktion in eine Gleitkommazahl uminterpretiert wird (1346), was zu einer Annäherung des Kehrwerts der ursprünglichen Gleitkommazahl führt.

3. Verfahren nach Anspruch 1 oder 2, wobei die kollaborative Sitzung innerhalb einer medizinischen Anwendung abläuft.

4. Verfahren nach einem der vorhergehenden Ansprüche, wobei die erste Rechenvorrichtung (101) zusammen mit den Segmentierungseingangsdaten (21) einen Hash-Wert der entsprechenden bearbeiteten Etikettenmaske (20) der zweiten Rechenvorrichtung (102) empfängt und ferner einen Hash-Wert für die jeweilige replizierte Etikettenmaske (10) berechnet, um die Korrektheit der Replikation der Etikettenmasken über die Rechenvorrichtungen hinweg sicherzustellen.

5. Verfahren nach einem der vorhergehenden Ansprüche, wobei das Aktualisieren unter Verwendung von mindestens einem Etiketten-Setzmodus durchgeführt wird, um die bestimmten Voxel in der replizierten Etikettenmaske auf den Etiketten-Identifikator der SegmentierungsEingangsdaten zu setzen, und einem Etiketten-Aufhebmodus, um einen spezifischen, früher gesetzten Etiketten-Identifikator von den bestimmten Voxeln in der replizierten Etikettenmaske zu entfernen.

6. Verfahren nach Anspruch 5, wobei das Aktualisieren unter Verwendung eines der folgenden Aktualisierungsmodi durchgeführt wird: Etikett freimachen, um alle Etiketten-Identifikatoren der bestimmten Voxel in der replizierten Etikettenmaske zurückzusetzen, Etiketten setzen für nicht zugeordnete Voxel, um den Etiketten-Identifikator der Segmentierungseingangsdaten in der replizierten Etikettenmaske nur für bestimmte Voxel, die noch nicht etikettiert sind, zu setzen, und Etiketten setzen für sichtbare Voxel, um den Etiketten-Identifikator der Segmentierungseingangsdaten in der replizierten Etikettenmaske nur für bestimmte Voxel, die gemäß einer angewandten Übertragungsfunktion sichtbar sind, zu setzen.

7. Verfahren nach einem der vorhergehenden Ansprüche, wobei für jedes Voxel der Etikettenmaske der Etiketten-Identifikator eines einzelnen Etiketts als 8-Bit-, 16-Bit- oder 32-Bit-Wert gespeichert wird.

8. Verfahren nach Anspruch 7, wobei für jedes Voxel der Etikettenmaske ein 8-Bit-, 16-Bit-, 32-Bit- oder längeres Bitfeld gespeichert wird, wobei jedes Bit einem Etikett entspricht, wodurch ermöglicht wird, für jedes Voxel eine Mehrzahl von Etiketten zu setzen.

9. Computerprogrammprodukt zum Replizieren einer dreidimensionalen Etikettenmaske (20) für einen dreidimensionalen Volumendatensatz (190) in einem Netzwerk (100) von Rechenvorrichtungen (101 bis 10n), wobei das Computerprogrammprodukt computerlesbare Anweisungen umfasst, die, wenn sie in einen Speicher einer Rechenvorrichtung geladen und von mindestens einem Prozessor der Rechenvorrichtung ausgeführt werden, bewirken, dass die Rechenvorrichtung das Verfahren nach einem der vorhergehenden Ansprüche durchführt.

10. Rechenvorrichtung (101) zum Replizieren einer dreidimensionalen Etikettenmaske (20) für einen dreidimensionalen Volumendatensatz (190) in einem Netzwerk (100) von Rechenvorrichtungen, umfassend:

eine Schnittstelle (110), die dazu eingerichtet ist, von dem Netzwerk von Rechenvorrichtungen einen Datensatz der aktuellen Etikettenmaske und des gesamten dreidimensionalen Volumendatensatzes (190) zu empfangen, wenn sie einer kollaborativen Sitzung in dem Netzwerk (100) zur Initialisierung der Rechenvorrichtung (101) beitritt, und die ferner dazu eingerichtet ist, eine Reihe von Segmentierungseingabedaten (21) zu empfangen, die von einer weiteren Rechenvorrichtung (102) des Netzwerks erzeugt werden, wobei die Reihe von Segmentierungseingabedaten eine Bearbeitung (2-1) der Etikettenmaske (20) auf der weiteren Rechenvorrichtung (102) beschreibt, wobei bestimmte Segmentierungseingabedaten aktuelle Positionsdaten, Größendaten und einen Etiketten-Identifikator umfassen, der einem kugelförmigen Segmentierungsobjekt (102-1) zugeordnet ist, das von der zweiten Rechenvorrichtung (102) zur interaktiven Bearbeitung der Etikettenmaske verwendet wird; ein Replikatormodul (101-10), das dazu eingerichtet ist, die bearbeitete Etikettenmaske der weiteren Rechenvorrichtung (102), die der Reihe von Segmentierungseingabedaten zugeordnet ist, zu replizieren, indem alle Segmentierungseingabedaten der Reihe mit den folgenden Modulen verarbeitet werden:

ein Ellipsoidmodul (101-11), das dazu eingerichtet ist, ein jeweiliges Ellipsoid zu berechnen, das dem kugelförmigen Segmentierungsobjekt (102-1) entspricht, das umgekehrt proportional zur Form der Voxel in der Etikettenmaske gestreckt ist;

ein Voxel-Identifizierungsmodul (101-12), das dazu eingerichtet ist, Voxel (vi) der Etikettenmaske, die eine Position innerhalb des Segmentierungsobjekts haben, zu bestimmen, und zwar basierend auf den Verhältnissen der Abstände zwischen einer jeweiligen Voxelposition und der Mittelpunktsposition des Ellipsoids in allen drei Dimensionen als Zähler, dividiert durch die jeweilige Halbachsenlänge des Ellipsoids als Nenner, wobei jede Halbachsenlänge eine Gleitkommazahl ist, die durch ganzzahlige Zahlen für Vorzeichen, Mantisse und Exponent dargestellt wird, wobei die Divisionen auf einer grafischen Verarbeitungseinheit der Rechenvorrichtung (101) durch Annäherung des Kehrwerts der jeweiligen Nenner durch Negieren der Exponenten der jeweiligen Halbachsenlängen unter Verwendung bitweiser Operationen und Multiplizieren des angenäherten Kehrwerts der jeweiligen Nenner mit ihren jeweiligen Zählern implementiert werden; und

ein Aktualisierungsmodul (101-13), das dazu eingerichtet ist, in der replizierten Etikettenmaske (10) die bestimmten Voxel gemäß dem Etiketten-Identifikator der Segmentierungseingangsdaten (21) zu aktualisieren.

11. Vorrichtung nach Anspruch 10, wobei der Voxel-Identifikator die bitweisen Divisionsoperationen für eine Division A/B durch Multiplizieren von A mit einer Annäherung von $B^{-1}$, das der Kehrwert von B ist, implementiert, wobei die Annäherung berechnet wird durch Subtrahieren von B, das als eine ganze Zahl interpretiert wird, von einer vorbestimmten ganzzahligen Konstante, die so vorbestimmt ist, dass das Ergebnis der ganzzahligen Subtraktion den Kehrwert der Gleitkommazahl durch Ausnutzen des standardisierten Kodierungsformats von Gleitkommazahlen, insbesondere der Position des Exponenten und des Bruches in der binären Darstellung, annähert, indem:

ein Compiler der Rechenvorrichtung angewiesen wird, die 32-Bit-Gleitkommazahl als ganze Zahl zu interpretieren, ohne die binäre Darstellung zu ändern,

die resultierende ganze Zahl von der vorbestimmten ganzzahligen Konstante subtrahiert wird und das Ergebnis der Subtraktion als Gleitkommazahl neu interpretiert wird, was eine Annäherung an den Kehrwert der ursprünglichen Gleitkommazahl ergibt.

12. Vorrichtung nach Anspruch 10 oder 11, wobei die kollaborative Sitzung eine Sitzung ist, die innerhalb einer medizinischen Anwendung ausgeführt wird.

13. Vorrichtung nach einem der Ansprüche 10 bis 12, wobei die Rechenvorrichtung (101) ferner dazu eingerichtet ist, zusammen mit den Segmentierungseingabedaten (21) einen Hash-Wert der entsprechenden bearbeiteten Etikettenmaske (20) der weiteren Rechenvorrichtung (102) zu empfangen und einen Hash-Wert für die jeweilige replizierte Etikettenmaske (10) zu berechnen, um die Korrektheit der Replikation der Etikettenmasken über die beiden Rechenvorrichtungen hinweg sicherzustellen.

14. Vorrichtung nach einem der Ansprüche 10 bis 13, wobei das Aktualisierungsmodul ferner dazu eingerichtet ist, einen beliebigen aus einer Mehrzahl von Aktualisierungsmodi zu unterstützen, die umfassen: einen Etiketten-Setzmodus, um die bestimmten Voxel in der replizierten Etikettenmaske auf den Etiketten-Identifikator der Segmentierungseingangsdaten zu setzen, einen Etiketten-Aufhebmodus, um einen spezifischen, früher gesetzten Etiketten-Identifikator von den bestimmten Voxeln in der replizierten Etikettenmaske zu entfernen, einen Etiketten-Freimachmodus, um alle Etiketten-Identifikatoren der bestimmten Voxel in der replizierten Etikettenmaske zurückzusetzen, einen Etiketten-Setzmodus für nicht zugeordnete Voxel, um den Etiketten-Identifikator der Segmentierungseingangsdaten in der replizierten Etikettenmaske nur für bestimmte Voxel, die noch nicht etikettiert sind, zu setzen, und einen Etiketten-Setzmodus für sichtbare Voxel, um den Etiketten-Identifikator der Segmentierungseingangsdaten in der replizierten Etikettenmaske nur für bestimmte Voxel, die gemäß einer angewendeten Übertragungsfunktion sichtbar sind, zu setzen.

15. Vorrichtung nach einem der Ansprüche 10 bis 14, wobei die Bits der Voxelwerte in der Etikettenmaske einen einzelnen ganzzahligen Etikettenwert für jedes Voxel darstellen, oder wobei jedes Bit der Voxelwerte in der Etikettenmaske einem separaten Etikett für das entsprechende Voxel entspricht.

**Revendications**

1. Procédé mis en oeuvre par ordinateur (1000) pour répliquer un masque d'étiquette tridimensionnel (20) pour un ensemble (190) de données de volume tridimensionnel dans un réseau (100) de dispositifs informatiques (101 à 10n), comprenant :

   lorsqu'un premier dispositif informatique (101) rejoint une session collaborative sur le réseau (100), l'initialisation (1100) du premier dispositif informatique (101) avec l'ensemble de données complet du masque d'étiquette actuel et l'ensemble (190) de données de volume tridimensionnel ;
   la réception (1200), au niveau du premier dispositif informatique (101), d'une série de données (21) d'entrée de segmentation générées par un deuxième dispositif informatique (102), où des données d'entrée de segmentation particulières comprennent des données de position actuelle, des données de taille et un identifiant d'étiquette associés à un objet (102-1) de segmentation en forme de sphère utilisés par le deuxième dispositif informatique (102) pour éditer de manière interactive le masque d'étiquette ;
   la réplication (1300), au niveau du premier dispositif informatique (101), du masque d'étiquette édité du deuxième dispositif informatique (102) associé à la série de données d'entrée de segmentation par : pour chacune des données d'entrée de segmentation de la série,

      calcul (1320) d'un ellipsoïde respectif qui correspond à l'objet (102-1) de segmentation en forme de sphère étiré de manière inversement proportionnelle à la forme des voxels dans le masque d'étiquette ;
      détermination (1340) des voxels (vi) du masque d'étiquette ayant une position à l'intérieur de l'objet de segmentation sur la base des rapports des distances entre une position de voxel respective et la position centrale de l'ellipsoïde dans les trois dimensions comme numérateurs divisées par la longueur de demi-axe respective de l'ellipsoïde comme dénominateurs, chaque longueur de demi-axe étant un nombre à virgule flottante représenté par des nombres entiers pour le signe, le significand et l'exposant, les divisions étant exécutées sur une unité de traitement graphique du premier dispositif informatique (101) par l'approximation de l'inverse multiplicatif des dénominateurs respectifs en annulant les exposants des longueurs des demi-axes respectives à l'aide d'opérations bit à bit, et en multipliant l'inverse multiplicatif approximatif des dénominateurs respectifs par leurs numérateurs respectifs ;
      et mise à jour (1360), dans le masque (10) d'étiquette répliqué, des voxels déterminés conformément à l'identifiant d'étiquette des données (21) d'entrée de segmentation.

2. Procédé selon la revendication 1, dans lequel les opérations de division bit à bit mettent en oeuvre une division A/B en multipliant A par une approximation de $B^{-1}$, étant l'inverse de B, dans lequel ladite approximation est calculée en soustrayant B, interprété comme un nombre entier, d'une constante entière prédéterminée, en ce que :

   un compilateur du premier dispositif informatique reçoit pour instruction d'interpréter (1342) le nombre flottant de 32 bits comme un nombre entier sans modifier la représentation binaire,
   l'entier résultant est soustrait (1344) de la constante entière prédéterminée, étant prédéterminée de sorte que le résultat de ladite soustraction entière se rapproche de l'inverse du nombre flottant en exploitant le format de codage standardisé des flottants, en particulier l'emplacement de l'exposant et la fraction dans la représentation binaire, et
   le résultat de la soustraction est réinterprété (1346) comme un nombre flottant, ce qui donne une approximation de l'inverse du nombre flottant d'origine.

3. Procédé selon la revendication 1 ou 2, dans lequel la session collaborative s'exécute dans une application médicale.

4. Procédé selon l'une quelconque des revendications précédentes, dans lequel le premier dispositif informatique (101) reçoit, conjointement avec les données (21) d'entrée de segmentation, une valeur de hachage du masque (20) d'étiquette édité correspondant du deuxième dispositif informatique (102), et calcule en outre une valeur de hachage pour le masque (10) d'étiquette répliqué respectif afin de garantir l'exactitude de la réplication des masques d'étiquette sur les dispositifs informatiques.

5. Procédé selon l'une quelconque des revendications précédentes, dans lequel la mise à jour est effectuée en utilisant au moins un mode set-label pour définir les voxels déterminés dans le masque d'étiquette répliqué sur l'identifiant d'étiquette des données d'entrée de segmentation, et un mode unset-label pour supprimer un identifiant d'étiquette spécifique défini précédemment à partir des voxels déterminés dans le masque d'étiquette répliqué.

**6.** Procédé selon la revendication 5, dans lequel la mise à jour est effectuée en utilisant l'un quelconque des modes de mise à jour suivants : clear-label pour réinitialiser tous les identifiants d'étiquette des voxels déterminés dans le masque d'étiquette répliqué, set-label-for-unassigned-voxels pour définir l'identifiant d'étiquette des données d'entrée de segmentation dans le masque d'étiquette répliqué uniquement pour les voxels déterminés qui ne sont pas encore étiquetés, et set-label-for-visible-voxels pour définir l'identifiant d'étiquette des données d'entrée de segmentation dans le masque d'étiquette répliqué uniquement pour les voxels déterminés qui sont visibles conformément à une fonction de transfert appliquée.

**7.** Procédé selon l'une quelconque des revendications précédentes, dans lequel, pour chaque voxel du masque d'étiquette, l'identifiant d'étiquette d'une étiquette unique est stocké sous la forme d'une valeur de 8 bits, 16 bits ou 32 bits.

**8.** Procédé selon la revendication 7, dans lequel, pour chaque voxel du masque d'étiquette, un champ de bits de 8 bits, 16 bits, 32 bits ou plus est stocké, où chaque bit correspond à une étiquette, permettant ainsi à une pluralité d'étiquettes d'être définies pour chaque voxel.

**9.** Produit-programme informatique pour répliquer un masque d'étiquette tridimensionnel (20) pour un ensemble (190) de données de volume tridimensionnel dans un réseau (100) de dispositifs informatiques (101 à 10n), le produit-programme informatique comprenant des instructions lisibles par ordinateur qui, lorsqu'il est chargé dans une mémoire d'un dispositif informatique et exécuté par au moins un processeur du dispositif informatique, amènent le dispositif informatique à réaliser le procédé selon l'une quelconque des revendications précédentes.

**10.** Dispositif informatique (101) pour répliquer un masque d'étiquette tridimensionnel (20) pour un ensemble (190) de données de volume tridimensionnel dans un réseau (100) de dispositifs informatiques, comprenant :

une interface (110) adaptée pour recevoir, du réseau de dispositifs informatiques, un ensemble de données du masque d'étiquette actuel et l'ensemble (190) complet de données de volume tridimensionnel lors de la participation à une session collaborative sur le réseau (100) pour initialiser le dispositif informatique (101), et en outre adaptée pour recevoir une série de données (21) d'entrée de segmentation générées par un autre dispositif informatique (102) du réseau, la série de données d'entrée de segmentation décrivant une édition (2-1) du masque d'étiquette (20) sur l'autre dispositif informatique (102), où des données d'entrée de segmentation particulières comprennent des données de position actuelle, des données de taille et un identifiant d'étiquette associés à un objet (102-1) de segmentation en forme de sphère utilisés par le deuxième dispositif informatique (102) pour éditer de manière interactive le masque d'étiquette ;
un module réplicateur (101-10) adapté pour répliquer le masque d'étiquette édité de l'autre dispositif informatique (102) associé à la série de données d'entrée de segmentation en traitant chaque donnée d'entrée de segmentation de la série avec les modules suivants :

un module ellipsoïde (101-11) adapté pour calculer un ellipsoïde respectif qui correspond à l'objet (102-1) de segmentation en forme de sphère étiré de manière inversement proportionnelle à la forme des voxels dans le masque d'étiquette ;
un module d'identification de voxel (101-12) adapté pour déterminer des voxels (vi) du masque d'étiquette ayant une position à l'intérieur de l'objet de segmentation sur la base des rapports des distances entre une position de voxel respective et la position centrale de l'ellipsoïde dans les trois dimensions comme numérateurs divisées par la longueur de demi-axe respective de l'ellipsoïde comme dénominateurs, chaque longueur de demi-axe étant un nombre à virgule flottante représenté par des nombres entiers pour le signe, le significand et l'exposant, les divisions étant mises en oeuvre sur une unité de traitement graphique du dispositif informatique (101) par l'approximation de l'inverse multiplicatif des dénominateurs respectifs en annulant les exposants des longueurs des demi-axes respectives à l'aide d'opérations bit à bit, et en multipliant l'inverse multiplicatif approximatif des dénominateurs respectifs par leurs numérateurs respectifs ; et
un module de mise à jour (101-13) adapté pour mettre à jour, dans le masque (10) d'étiquette répliqué, des voxels déterminés conformément à l'identifiant d'étiquette des données (21) d'entrée de segmentation.

**11.** Dispositif selon la revendication 10, dans lequel l'identifiant de voxel met en oeuvre les opérations de division bit à bit pour une division A/B en multipliant A par une approximation de $B^{-1}$, étant l'inverse de B, dans lequel ladite approximation est calculée en soustrayant B, interprété comme un nombre entier, d'une constante entière prédéterminée, étant prédéterminée de sorte que le résultat de ladite soustraction entière se rapproche de l'inverse du nombre flottant en exploitant le format de codage standardisé des flottants, en particulier l'emplacement de l'exposant et de la fraction dans la représentation binaire, en ce que :

un compilateur du dispositif informatique reçoit pour instruction d'interpréter le nombre flottant de 32 bits comme un nombre entier sans modifier la représentation binaire,
le nombre entier résultant est soustrait de la constante entière prédéterminée, et
le résultat de la soustraction est réinterprété comme un nombre flottant, ce qui donne une approximation de l'inverse du nombre flottant d'origine.

12. Dispositif selon la revendication 10 ou 11, dans lequel la session collaborative est une session exécutée dans une application médicale.

13. Dispositif selon l'une quelconque des revendications 10 à 12, dans lequel le dispositif informatique (101) est en outre adapté pour recevoir, conjointement avec les données (21) d'entrée de segmentation, une valeur de hachage du masque (20) d'étiquette édité correspondant de l'autre dispositif informatique (102), et pour calculer une valeur de hachage pour le masque (10) d'étiquette répliqué respectif afin de garantir l'exactitude de la réplication des masques d'étiquette sur les deux dispositifs informatiques.

14. Dispositif selon l'une quelconque des revendications 10 à 13, dans lequel le module de mise à jour est en outre adapté pour prendre en charge l'un quelconque d'une pluralité de modes de mise à jour comprenant : un mode set-label pour définir les voxels déterminés dans le masque d'étiquette répliqué sur l'identifiant d'étiquette des données d'entrée de segmentation, un mode unset-label pour supprimer un identifiant d'étiquette spécifique défini précédemment des voxels déterminés dans le masque d'étiquette répliqué, un mode clear-label pour réinitialiser tous les identifiants d'étiquette des voxels déterminés dans le masque d'étiquette répliqué, un mode set-label-for-unassigned-voxels pour définir l'identifiant d'étiquette des données d'entrée de segmentation dans le masque d'étiquette répliqué uniquement pour les voxels déterminés qui ne sont pas encore étiquetés, et un mode set-label-for-visible-voxels pour définir l'identifiant d'étiquette des données d'entrée de segmentation dans le masque d'étiquette répliqué uniquement pour les voxels déterminés qui sont visibles conformément à une fonction de transfert appliquée.

15. Dispositif selon l'une quelconque des revendications 10 à 14, dans lequel les bits des valeurs de voxel dans le masque d'étiquette représentent une valeur d'étiquette entière unique pour chaque voxel, ou dans lequel chaque bit des valeurs de voxel dans le masque d'étiquette correspond à une étiquette distincte pour le voxel correspondant.

FIG. 1

initializing first computing device with
dataset of current label mask 1100

1000

receiving segmentation input 1200

replicating edited label mask of second
computing device associated with
segmentation input data 1300

computing ellipsoid from
segmentation input data 1320

determining voxels inside
segmentation object 1340

updating replicated label mask in
with label identifier 1360

FIG. 2A

1340

interpreting float number as integer
number without changing binary
representation 1342

subtracting integer from
predetermined integer constant
1344

FIG. 2B

reinterpreting subtraction result as
float number 1346

FIG. 3A

FIG. 3B

FIG. 3C

FIG. 4A

FIG. 4B

FIG. 4C

sign    exponent (8 bits)                    fraction (23 bits)                    500

bit index: 31 30                          23 22                                            0

FIG. 5

610

FIG. 6A    ▨  vi (voxels inside segmentation object)

620

FIG. 6B

700

FIG. 7

800

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2022346888 A1
- US 2020054398 A1

**Non-patent literature cited in the description**

- **CHHEANG VUTHEA et al.** A Collaborative Virtual Reality Environment for Liver Surgery Planning. *Computers & Graphics,* July 2021, vol. 99 (6
- **BEICHEL REINHARD et al.** Liver segmentation in contrast enhanced CT data using graph cuts and interactive 3D segmentation refinement methods. *Med Phys.,* 21 February 2012, vol. 39 (3), 1361-1373
- IEEE Standard for Floating-Point Arithmetic. *IEEE Std 754-2019 (Revision of IEEE 754-2008,* 22 July 2019, 1-84
- **WHITE, S. ; COULTER, D. ; JACOBS, M. ; SATRAN, M.** Floating-point rules (Direct3D 11). *Programming Guide for Direct3D 11, Microsoft,* 2020, https://learn.microsoft.com/en-us/windows/win32/direct3d11/floating-point-rules